# EUROPEAN PATENT SPECIFICATION

(11) **EP 2 480 203 B1**
(45) Date of publication and mention of the grant of the patent: **24.08.2016**
(21) Application number: 10773176.2
(22) Date of filing: 22.09.2010
(51) Int. Cl.: A61K 9/00, A61K 9/14, A61K 31/167, A61K 31/35, A61M 15/00

(54) **DRY POWDER FORMULATION OF TIOTROPIUM CARRIED IN BLISTER STRIP**
IN EINEM BLISTERSTREIFEN GETRAGENE TROCKENPULVERFORMULIERUNG AUS TIOTROPIUM
FORMULATION DE POUDRE SÈCHE DE TIOTROPIUM CONTENUE DANS UNE BANDE ALVÉOLÉE THERMOFORMÉE

(30) Priority: 23.09.2009 TR 200907236
(43) Date of publication of application: 01.08.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000183
(87) International publication number: WO 2011/037549

(56) References cited:
- EP-A1- 0 703 157
- WO-A1-00/47200
- WO-A1-2004/011070
- WO-A1-2005/053647
- WO-A1-2007/012871
- WO-A2-2008/102128
- US-A1- 2005 042 174
- US-A1- 2005 121 032
- US-A1- 2008 197 045

## Description

The present invention relates to a method for providing effective inhalation for the treatment of respiratory diseases, in which the medicament formulation containing tiotropium, which is in dry powder form, is delivered to the lungs from blisters arranged adjacently in a peelable blister strip.

Tiotropium (Formula I) is an anticholinergic agent with chemical name (1α,2β,4β,7β)- 7-[(hydroxidi-2-thienylacetyl)oxy]-9,9-dimethyl-3-oxa-9-azoniatricyclo[3.3.1.0^{2,4}]nonane.

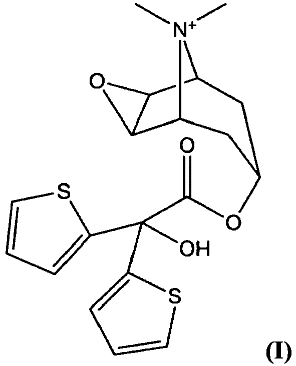

Tiotropium is described in European patent application numbered EP0418716 for the first time. In this patent document; processes for preparing tiotropium, pharmaceutical compositions containing tiotropium, long-acting, strong anticholinergic activity of tiotropium and use of it in the treatment of respiratory disorders, are defined.

Tiotropium antagonises effect of acetylcholine via blocking cholinergic muscarinic receptor. Tiotropium is separated slowly from M1 and M3 receptors that cause broncho-construction, and it is separated rapidly from M2 receptors that inhibit release of acetylcholine from cholinergic nerve endings. This situation occurred in lung receptors demonstrates long acting bronchodilator activity of the drug. Additionally, tiotropium is a long-acting, strong, anticholinergic bronchodilator which is administered orally for the treatment of respiratory disorders.

Tiotropium is a commonly preferred anticholinergic, because of showing long-acting action for the treatment of respiratory disorders especially chronic disorders such as asthma and chronic obstructive pulmonary disorder (COPD) which threaten the society widely. The inhalation product for the treatment of these disorders is sold by Boehringer and Pfizer under the trade name Spiriva® Handihaler®. This product contains Handihaler® which is a single-dose inhaler and Spiriva® which contains dry powder formulation of tiotropium bromide monohydrate and consists of single dose dry powder inhaler capsules used only in the Handihaler®. This drug which is administered via inhalation route is given in lower doses compared to the the drug given via oral and parenteral route, and shows desired effect and less side-effects. Gastrointestinal disturbances, such as low resolution, low permeability, drug irritation, production of undesired metabolites and decrease of bioavailability depending on food, which are change the effectiveness of the drug, are felt in minimum level because of the fact that the drugs administered via inhalation route, reach directly to target area and do not get into circulation of blood.

Nevertheless, just before every inhalation, one capsule should be taken out of the blister pack and loaded to capsule chamber of a single dose dry powder inhaler. Since the capsule can be loaded into the capsule chamber in a non-hygienic way, there is a risk of inhaling foreign matters.

Additionally, maintaining the stability and preventing the agglomeration of dry powder formulation which is contained in a capsule that is prepared for use in a single dose dry powder inhaler in order to achieve an effective inhalation should be considered. Hence, the capsules, which are convenient for use in a single dose inhaler are carried in a blister pack to provide a secondary protection for maintaining the stability of the dry powder formulation in the capsule. This reduces the moisture transition significantly. Even though this method solves the problem of maintaining stability of the dry powder formulation, it is not cost-effective.

Moreover, the mechanical components, such as needles that are contained in a single dose dry powder inhalers with capsule are used for piercing capsule. These may become blunt or lose certain position over time. Thus, an efficient piercing can not be achieved. Since this reduce the rate of utilization of the dry powder formulation in the capsule, effective amount of the dry powder formulation is not delivered to the lungs. Additionally, there is a risk of inhaling the plastic parts of the capsule which stick to the edge of piercing components of the inhaler during the piercing operation.

As an alternative to the inhalation of the dry powder formulation from the capsule, there are different inhalation methods such as the inhalation of a formulation containing one or more active substances from the metered dose inhalers, the nebulizers or the dry powder inhaler containing blister packs pierced just before inhalation. Nebulization and aerosol formulations contain the active substances in liquid form. It is more difficult to provide stability and dose accuracy in liquid form formulations compared to the dry powder formulations. The blister packs pierced just before inhalation suffer from some drawbacks which are caused by the piercing components in the multi dose dry powder inhalers as mentioned before. Additionally, the metered dose inhaler requires the mechanical components to be contained in this inhaler for an effective and sufficient inhalation of the dry powder formulation from the reservoir. Thus, these mechanic components cause increase in the size and weight of the inhaler and difficulty of use. Furthermore, in all of these methods, problems caused by the formulation being in the liquid form and piercing operation of the capsule or a blister pack in the inhaler, lead to drawbacks related to the stability and as a result of that reliability of the drug formulation.

Because of the above mentioned reasons, there is a need to develop a method which provides the inhalation of the dry powder formulation containing tiotropium to provide controlled dosing of the dry powder formulation and maintain the stability of the dry powder formulation in an user-friendly, hygenic, cost effective way.

Suprisingly, the inventor has found that the dry powder formulation which contains tiotropium, is inhaled effectively from the blisters which are arranged adjacently on a peelable blister strip and meets the requirements mentioned above.

In one aspect, the present invention provides the dry powder formulation which contains tiotropium and is inhaled from the blisters of the peelable blister strip.

In another aspect, the present invention provides the dry powder formulation which contains tiotropium and inhaled by providing controlled dosing of the dry powder formulation in an effective and stable way.

In another aspect, the present invention provides the dry powder formulation which contains tiotropium and is inhaled in an effective, hygienic, user-friendly way.

In another aspect, the present invention provides a method for inhaling the dry powder formulation, in which the dry powder formulation containing tiotropium is inhaled from the blisters of the peelable blister strip.

In another aspect, the present invention provides the medicament formulation which contains tiotropium and is inhaled from a multi-dose inhaler.

The medicament formulation in accordance with the present invention contains optionally one or more excipients.

According to the invention, excipient used in said dry powder formulation can be selected from the group comprising monosaccharides, disaccharides, polysaccharides, and oligosaccharides. Preferably lactose is used.

According to the invention, said dry powder formulation contains the excipient in an amount in the range of 0 to 50 mg.

According to the invention, the cavity volume of the blisters of the blister strip, which carry and store said dry powder formulation containing tiotropium, is in the range of 22-23 mm³.

According to the invention, the blister strip which consists of blisters which have a cavity volume in the range of 22 to 23 is used for providing efficient inhalation by dealing with the drawbacks defined herein before. Additionally, each blister cavity having the volume described above is filled up to 70-100 %, preferably up to 90-100 % of the said volume.

The peelable blister strip wherein the blisters are arranged adjacently consists of a lid sheet and a base sheet which are closed very tightly to provide impermeability by using any suitable method.

The lid sheet and the base sheet of the peelable blister strip, consists of many layers such as polymeric layer, aluminum foil and optionally Aclar® fluoropolimer film. Preferably, Aclar® fluoropolimer film is between the layers which make up the lid sheet and the base sheet of the blister strip.

Aclar® fluoropolymer film is a polymeric film which is used for production of the blister strip and provides high moisture protection. This chemically inert film does not cause any change in taste of the formulation when it is in contact with the dry powder formulation. It easily forms a lamellar structure with other polymeric layers which are made from various polymers. It is suitable for treatment with heat.

Desiccant agents are optionally added to the polymeric layers in order to reduce moisture and gas permeability of the polymeric layers for protection of stability of the dry powder formulation contained in the blisters which are arranged adjacently. Some examples of the desiccant agents are silica gel, zeolite, alumina, baucsite, anhydrous calcium sulfate, activated carbon, clay capable of absorbing water.

According to the invention, aluminium foil is used both in the lid sheet and in the base sheet of the peelable blister strip to provide high humidity and gas protection because of that aluminium foil is conventionally used in both the lid sheet and the base sheet of the blister strip for high humidty and gas protection. These layers must have the sufficient thickness which provides the protection for the stability of humidity sensitive dry powder formulation which is stored in blister cavity. Because of this reason, the thickness of aluminium foil that is used in the lid sheet and the base sheet of the blister strip is in the range of 10 to 40 µm, preferably of 15 to 30 µm.

The polymeric layers which are contained in the lid sheet and the base sheet of the peelable blister strip in accordance with the present invention may be made from either same or different polymers. The thickness of these polymeric layers depends on the type of polymeric substance used and its properties. Therefore, the thickness of each polymeric layer which is used in the lid sheet and the base sheet of said blister strip is in the range of 15 to 60 µm, preferably of 20 to 35 µm depending on the type of polymer used.

The inside layer of blister cavity of said blister strip which is in contact with dry powder formulation is polymeric layer because of the fact that aluminium foil causes adhesion of a part of dry powder formulation to inside layer of the cavity due to electrostatic forces, and hence cause uncontrolled dosing.

According to the present invention, the polymers used for forming polymeric layers are preferably selected from a group comprising thermo-plastic polymers such as polyethylene, polypropylene, polystyrene, polyolefin, polyamide, polyvinyl chloride, polyurethane.

Moreover, said blisters which placed in the peelable blister strips, can be in any shape under the condition that mentioned above.

Said dry powder formulation which contains tiotropium, is decomposed by an amount of 3% with respect to the total weight of the formulation after 16 months under the condition that said dry powder formulation is stored in the blisters of the peelable blister strip, in the temperature of 25°C and in the relative humidity (RH) of %60.

In accordance with the present formulation, the active agents which is contained in the medicament formulation is tiotropium and it is selected from a group comprising its pharmaceutically acceptable salts, solvates and/or hydrates thereof. According to the invention, it is preferably tiotropium bromide anhydrate.

Said dry powder form, which is stored in the blister cavities of the peelable blister strip is produced with methods known in the prior art. The medicament formulation which is in dry powder form, contains the active agents with the particle size of less than 20 µm. Lactose is used as excipient. The excipients with fine and coarser particles which have different particle size range, are used in order to provide effective inhalation of the medicament formulation.

According to the invention, said dry powder formulation contains tiotropium or pharmaceutically acceptable salts which is present in the range of 1 to 50 µg in each blister of the peelable blister strip.

The medicament formulation in accordance with present invention can be used for the treatment of many respiratory diseases such as asthma, chronic obstructive pulmonary disorder (COPD) and allergic rhinitis. Accordingly, the respiratory diseases include but are not restricted to allergic or non-allergic asthma in various phases, acute lung injury (ALI), acute respiratory distress syndrome (ARDS), exacerbation of airways hyperactivity, bronchiectasis, chronic obstructive pulmonary, airways or lung diseases (COPD, COAD or COLD) including emphysema and chronic bronchitis, pneumoconiosis, aluminosis, anthracosis, asbestosis, chalicosis, ptilosis, siderosis, silicosis, tabacosis and byssinosis. The treatment of said diseases may be prophylactic or symptomatic. In addition to this, the medicament composition in accordance with the present invention is used especially for the symptomatic treatment of asthma, allergic rhinitis and COPD.

## Claims

1. A medicament formulation containing tiotropium or a pharmaceutically acceptable salt thereof for the treatment of respiratory disorders is wherein said medicament formulation is in dry powder form and is carried and stored in a peelable blister strip and is administered by dry powder inhaler **characterized in that**:
• each blister cavity is filled up to 70 to 100% of the total volume and is administered
• each blister has a cavity volume of 22 to 23 mm³.

2. A medicament formulation according to Claim 1 wherein said medicament formulation comprises optionally one or more pharmaceutically acceptable excipients.

3. A medicament formulation in dry powder form according to claim 2 wherein the excipient is selected form the group comprising monosaccharides, disaccharides, polysaccharides and oligosaccharides.

4. A medicament formulation in dry powder form according to claim 2 wherein the excipient is a disaccharide.

5. A medicament formulation in dry powder form according to claim 2 wherein the excipient is preferably lactose.

6. A medicament formulation in dry powder form according to claim 1 wherein lactose is present in an amount in the range of 0 to 50 µg.

7. A medicament formulation in dry powder form according to claim 1 wherein the active agents have average particle size of less than 20 µm.

8. A medicament formulation in dry powder form according to claim 1 wherein tiotropium or pharmaceutically acceptable salts thereof is present in an amount in the range of 1 to 50 µg

9. A medicament formulation in dry powder form according to claim 1 wherein tiotropium or pharmaceutically acceptable salts thereof is present in an amount in the range of 1 to 50 µg and one or more pharmaceutically acceptable excipients which is present in the range of 0 to 50 µg.

10. Use of tiotropium or a pharmaceutically acceptable for the preparation of a medicament formulation in dry powder form according to any of the preceding claims.

11. A medicament formulation in dry powder form according to any one of the preceding claims wherein the active agent is tiotropium bromide anhydrate.

12. A peelable blister strip according to claim 1 wherein each blister cavity is filled up to 90 to 100% of the total volume

## Patentansprüche

1. Eine Medikamentenformulierung, die Tiotropium oder ein pharmazeutisch verträgliches Salz davon zur Behandlung von Atemwegsstörungen enthält, wobei die Medikamentenformulierung in Trockenpulverform ist und in einem abziehbaren Blisterstreifen getragen und gelagert wird und durch Trockenpulver -Inhalator verabreicht wird, **dadurch gekennzeichnet, dass**:
• jede Blisterkavität auf 70 bis 100% des Gesamtvolumens gefüllt und verabreicht wird
• jede Blisterkavität ein Hohlraumvolumen von 22 bis 23 mm³ besitzt

2. Eine Medikamentenformulierung nach Anspruch 1, wobei die genannte Medikamentenformulierung gegebenenfalls einen oder mehrere pharmazeutisch annehmbaren Hilfsstoffe umfasst.

3. Eine Medikamentenformulierung in Trockenpulverform nach Anspruch 2, wobei der Hilfsstoff aus der Gruppe ausgewählt wird, die Monosaccharide, Disaccharide, Polysaccharide und Oligosaccharide umfasst.

4. Eine Medikamentenformulierung in Trockenpulverform nach Anspruch 2, wobei der Hilfsstoff ein Disaccharid ist.

5. Eine Medikamentenformulierung in Trockenpulverform nach Anspruch 2, wobei der Hilfsstoff vorzugsweise Lactose ist.

6. Eine Medikamentenformulierung in Trockenpulverform nach Anspruch 1, wobei Lactose in einer Menge im Bereich von 0 bis 50 µg vorhanden ist.

7. Eine Medikamentenformulierung in Trockenpulverform nach Anspruch 1, wobei die aktiven Mittel durchschnittliche Partikelgröße von weniger als 20 µm haben

8. Eine Medikamentenformulierung in Trockenpulverform nach Anspruch 1, wobei Tiotropium oder deren pharmazeutisch annehmbaren Salze in einer Menge im Bereich von 1 bis 50 µg vorhanden ist

9. Eine Medikamentenformulierung in Trockenpulverform nach Anspruch 1, wobei Tiotropium oder deren pharmazeutisch annehmbaren Salze in einer Menge im Bereich von 1 bis 50 µg vorhanden ist und ein oder mehrere pharmazeutisch annehmbaren Hilfsstoffe im Bereich von 0 bis 50 µg vorhanden ist

10. Verwendung von Tiotropium oder eines pharmazeutisch annehmbaren zur Herstellung einer Medikamentenformulierung in Trockenpulverform nach einem der vorhergehenden Ansprüche.

11. Eine Medikamentenformulierung in Trockenpulverform nach einem der vorhergehenden Ansprüche, wobei der Wirkstoff Tiotropiumbromid-Anhydrat ist.

12. Ein abziehbarer Blisterstreifen nach Anspruch 1, wobei jede Blisterkavität auf 90 bis 100% des Gesamtvolumens aufgefüllt wird

## Revendications

1. Une formulation de médicament contenant du tiotropium ou celui-ci acceptable d'un sel pharmaceutiquement pour le traitement de maladies respiratoires est dans lequel ladite formulation de médicament est sous forme de poudre sèche et est transporté et stocké dans une bande de blister pelable et est administré par l'inhalateur à poudre sèche caractérisé dans lequel :
• chaque cavité d'ampoule est remplie jusqu'à 70 à 100% du volume total et est administré
• chaque cloque présente un volume de cavité de 22 à 23 mm³

2. Formulation de médicament selon la revendication 1, dans lequel ladite formulation de médicament comprend optionellement un ou plusieurs excipients pharmaceutiquement acceptables,

3. Une formulation de médicament sous forme de poudre sèche selon la revendication 2, dans lequel l' excipient est choisi forme le groupe comprenant les monosaccharides, les disaccharides, les polysaccharides et oligosaccharides.

4. Formulation de médicament sous forme de poudre sèche selon la revendication 2, dans lequel l'excipient est un disaccharide.

5. Formulation de médicament sous forme de poudre sèche selon la revendication 2, dans lequel l'excipient est de préférence le lactose.

6. Formulation de médicament sous forme de poudre sèche selon la revendication 1, dans lequel le lactose est présent en une quantité comprise dans l'intervalle de 0 à 50µg

7. Formulation de médicament sous forme de poudre sèche selon la revendication 1, dans lequel les agents actifs ont une taille moyenne inférieure à 20 µm

8. Formulation de médicament sous forme de poudre sèche selon la revendication 1, dans lequel le tiotropium ou les sels pharmaceutiquement acceptables de celui-ci est présent en une quantité comprise dans l'intervalle de 1 à 50 µg

9. Une formulation de médicament sous forme de poudre sèche selon la revendication 1, dans lequel tiotropium ou de sels pharmaceutiquement acceptables est présent en une quantité dans l'intervalle de 1 à 50 µg et un ou plusieurs excipients pharmaceutiquement acceptables qui est présent dans l'intervalle de 0 à 50 µg

10. Utilisation du tiotropium ou pharmaceutiquement acceptable pour la préparation d'une formulation de médicament sous forme de poudre sèche selon l'une des revendications précédentes.

11. Formulation de médicament sous forme de poudre sèche selon l'une des revendications précédentes, dans lequel l'agent actif est le bromure de tiotropium anhydrate.

12. Une bande de blisters pelables selon la revendication 1, dans lequel chaque cavité de la coque est remplie jusqu'à 90 à 100% du volume total
